# EUROPÄISCHE PATENTSCHRIFT

(11) **EP 3 906 902 B1**
(45) Veröffentlichungstag und Bekanntmachung des Hinweises auf die Patenterteilung: **16.11.2022**
(21) Anmeldenummer: 21171919.0
(22) Anmeldetag: 04.05.2021
(51) Int. Cl.: A61F 7/02

(54) **SYSTEM ZUR TEMPERIERUNG DES KOPFES EINER PERSON**
SYSTEM FOR TEMPERATURE CONTROL OF THE HEAD OF A PERSON
SYSTÈME DE CLIMATISATION POUR LA TÊTE D'UNE PERSONNE

(30) Priorität: 06.05.2020 DE 202020001965 U
(43) Veröffentlichungstag der Anmeldung: 10.11.2021
(73) Patentinhaber: Hilotherm Holding AG, 6317 Oberwil bei Zug (CH)
(72) Erfinder: Stegmann, Christian, 87545 Burgberg im Allgäu (DE)
(74) Vertreter: Wallinger, Michael

(56) Entgegenhaltungen:
- EP-A1- 3 069 699
- US-A1- 2012 130 457
- US-A1- 2014 236 271
- US-A1- 2016 317 348
- US-A1- 2016 354 232
- US-A1- 2018 311 070

## Beschreibung

Die vorliegende Erfindung betrifft ein System zur Temperierung des Kopfes einer Person.

Unter einer Temperierung wird dabei eine Kühlung oder eine Erwärmung auf eine bestimmte Temperatur verstanden, wobei diese Temperatur vorzugsweise möglichst konstant gehalten werden soll.

Eine Kühlung des Kopfes kann insbesondere genutzt werden, um bei Operationen während einer Narkose den Sauerstoffbedarf des Gehirns zu reduzieren und somit negative Spätfolgen durch eine narkosebedingte Sauerstoffunterversorgung des Gehirns zu reduzieren. Ebenso kann eine passend ausgewählte Temperierung des Kopfes Kopfschmerzen, insbesondere Migräneschmerzen, lindern.

EP 1 333 786 offenbart eine Kopf- und Halsmanschette zur Kühlung des Kopfes zur Traumabehandlung, um eine Schwellung des Gehirns zu minimieren. Eine Schwellung des Gehirns führt zu einer unzureichenden Blut- und somit auch Sauerstoffversorgung. Die Kopfmanschette besteht aus mehreren individuellen Teilen, um eine gute Abdeckung des Kopfes zu erreichen.

US 2016/0317348 A1 beschreibt eine Kühlkappe, die zum Anschließen an eine Kühleinheit zum Zirkulieren von gekühltem Fluid durch die Kappe angepasst ist. Die Kappe ist aus mindestens drei Materialschichten gebildet, die um ihren Umfang herum dichtend angebracht sind, um eine Kühlfluidkammer zu bilden, wobei die Kappe konfiguriert ist, um Kompression und Kühlung auf die Kopfhaut auszuüben.

US 2016/0354232 A1 beschreibt ein Kühlkissen und ein System für die Patientenversorgung. Das Kühlkissen umfasst eine obere Kammer, eine untere Kammer und eine Zwischenkammer, die zwischen der oberen Kammer und der unteren Kammer angeordnet ist.

US 2018/0311070 A1 beschreibt ein Kühlkissen zur Verwendung in einem nichtinvasiven medizinischen Kühlverfahren zum Kühlen mindestens eines Teils des Körpers einer Person mittels eines Kühlfluids, das im Gebrauch durch das Kühlkissen fließt. Das Kühlkissen besteht aus einem unteren Blatt und einem oberen Blatt und besteht aus flexiblem und flüssigkeitsdichtem Material.

EP 3 069 699 A1 betrifft eine Wärmebehandlungsvorrichtung zur Verwendung bei der wärmetherapeutischen Behandlung eines menschlichen Körperteils mit einem Wärmeübertragungskörper mit zwei flexiblen Materialschichten, die zwischen sich einen oder mehrere von einem wärmeübertragenden Fluid durchströmbare Strömungsräume definieren, und mindestens eine erste Zuleitung zum Zuführen und mindestens eine zweite Zuleitung zum Abführen eines Wärmeübertragungsfluids zu bzw. von dem Wärmeübertragungskörper.

Der vorliegenden Erfindung liegt die Aufgabe zugrunde, ein verbessertes System zur Temperierung des Kopfes einer Person bereitzustellen.

Die Lösung dieser Aufgabe wird gemäß der Lehre des unabhängigen Anspruchs 1 erreicht. Verschiedene Ausführungsformen und Weiterbildungen der Erfindung sind Gegenstand der Unteransprüche.

Die Erfindung betrifft ein System zur Temperierung des Kopfes einer Person. Das System weist auf:
(i) einen zentralen Abschnitt, welcher eingerichtet ist, wenigstens einen Teil der Oberseite des Kopfes zu bedecken;
(ii) wenigstens einen frontalen Abschnitt, welcher eingerichtet ist, wenigstens einen Teil der Stirn der Person zu bedecken;
(iii) wenigstens einen rechten Abschnitt, welcher eingerichtet ist, wenigstens einen Teil der rechten Seite des Kopfes der Person zu bedecken;
(iv) wenigstens einen linken Abschnitt, welcher eingerichtet ist, wenigstens einen Teil der linken Seite des Kopfes der Person zu bedecken; und
(v) wenigstens einen rückseitigen Abschnitt, welcher eingerichtet ist, wenigstens einen Teil des Hinterkopfes der Person zu bedecken;

wobei jeder einzelne Abschnitt eingerichtet ist, von einem Temperierungsfluid, welches eingerichtet ist, den Kopf zu temperieren, durchflossen zu werden;
wobei jeder einzelne Abschnitt mit wenigstens einem weiteren Abschnitt verbunden ist und in einer Fluidverbindung für das Temperierungsfluid mit diesem weiteren Abschnitt steht;
wobei wenigstens ein Abschnitt eine Einflussöffnung und wenigstens ein Abschnitt eine Abflussöffnung für das Temperierungsfluid aufweist,
wobei das System zur Temperierung wenigstens ein Verbindungselement aufweist, welches eingerichtet ist, wenigstens zwei Abschnitte teilweise trennbar aneinander zu befestigen, und wobei die Form des Systems durch die teilweise trennbare Befestigung der wenigstens zwei Abschnitte aneinander an die Form des Kopfes der Person anpassbar ist, dadurch gekennzeichnet, dass der linke Abschnitt, der rechte Abschnitt und der rückseitige Abschnitt wenigstens zwei zueinander bewegbare Unterabschnitte aufweist, welche, wie auch der frontale Abschnitt (12), jeweils mit dem zentralen Abschnitt verbunden sind und in einer Fluidverbindung für das Temperierungsfluid mit dem zentralen Abschnitt stehen, und wobei das System eine im Wesentlichen sternförmige Anordnung des frontalen Abschnitts, des linken Abschnitts mit den beiden linken Unterabschnitten, des rechten Abschnitts mit den beiden rechten Unterabschnitten sowie des rückseitigen Abschnitts mit den beiden rückseitigen Unterabschnitten an dem zentralen Abschnitt aufweist.

Unter einem "Temperierungsfluid" ist eine Flüssigkeit, ein Gel und/oder ein Gas zu verstehen, welches eingerichtet ist, Energie in Form von Wärme an den Kopf der Person über den jeweils durchflossenen Abschnitt abzugeben bzw. von dem Kopf der Person aufzunehmen.

Unter einer "Einflussöffnung" ist eine Öffnung zu verstehen, durch die das Temperierungsfluid in das System einfließen kann. Unter einer "Abflussöffnung" ist eine Öffnung zu verstehen, durch die das Temperierungsfluid aus dem System abfließen kann. Eine Einflussöffnung kann insbesondere auch eine Abflussöffnung sein. Die Öffnungen können insbesondere, beispielsweise in einem Nichtgebrauchs- oder Lagerzustand des Systems, verschlossen werden.

Das System weist vorzugsweise eine Oberseite und eine Unterseite auf. Diese bestehen vorzugsweise aus zwei aufeinanderliegenden Lagen aus einem flexiblen Material, insbesondere aus einer Kunststofffolie. Die Oberseite und die Unterseite sind vorzugsweise an ihren Rändern umlaufend und fluiddicht miteinander verbunden, insbesondere verschweißt. Dadurch entsteht zwischen den beiden Lagen ein Hohlraum, welcher von dem Temperierungsfluid durchflossen werden kann. Die einzelnen Abschnitte des Systems kommen vorzugsweise durch die Abteilung einzelner Bereiche des Systems zustande, insbesondere durch Einschnürungen des Randes der miteinander verbundenen Ober- und Unterseite.

Mithilfe des vorgenannten Systems nach dem ersten Aspekt lässt sich die Temperatur des Kopfes der Person regulieren. Durch die einzelnen Abschnitte des Systems werden die diesen jeweils zugeordneten Abschnitte des Kopfes temperiert, und alle Abschnitte können durch die Fluidverbindungen zwischen den Abschnitten des Systems simultan mit dem Temperierungsfluid versorgt werden.

Nachfolgend werden bevorzugte Ausführungsformen des Systems beschrieben, die jeweils, soweit dies nicht ausdrücklich ausgeschlossen wird oder technisch unmöglich ist, beliebig miteinander kombiniert werden können.

Gemäß einer bevorzugten Ausführungsform sind der frontale Abschnitt, der linke Abschnitt, der rechte Abschnitt und der rückseitige Abschnitt mit dem zentralen Abschnitt verbunden und stehen in einer Fluidverbindung für das Temperierungsfluid mit dem zentralen Abschnitt.

Eine Verbindung der genannten Abschnitte mit dem zentralen Abschnitt hat den Vorteil, dass Verbindungsstellen weniger stark belastet werden, da keine Aneinanderkettung einzelner Abschnitte vorliegt, wodurch auf jeder Verbindungsstelle lediglich die Gewichtskraft eines Abschnittes lastet. Weiter sorgt eine Verbindung mit dem zentralen Abschnitt für eine bessere Auflage der jeweiligen Abschnitte an dem Kopf und für eine mögliche Reduzierung ansonsten nötiger Fixierungshilfen, da der zentrale Abschnitt bei einer aufrechten Position des Kopfes auf dem Kopf aufliegt und durch die Gravitation bereits gehalten werden kann.

Gemäß der Erfindung weist der linke Abschnitt, der rechte Abschnitt und der rückseitige Abschnitt wenigstens zwei zueinander bewegbare Unterabschnitte auf, welche jeweils mit dem zentralen Abschnitt verbunden sind und in einer Fluidverbindung für das Temperierungsfluid mit dem zentralen Abschnitt stehen.

Eine Unterteilung eines oder mehrerer Abschnitte in mehrere Unterabschnitte hat den Vorteil, dass die jeweiligen Unterabschnitte besser zueinander bewegbar sind und somit ein besserer Kontakt mit dem jeweiligen Kopfbereich stattfinden kann.

Gemäß einer bevorzugten Ausführungsform durchfließt das Temperierungsfluid nach Eintritt durch die Einflussöffnung auf dem Weg zur Abflussöffnung jeden Abschnitt des Systems.

Indem jeder einzelne Abschnitt des Systems durchflossen wird, wird verhindert, dass Bereiche nicht durchflossen werden und die Temperierleistung des Fluids in diesen Bereichen nachlässt.

Gemäß einer bevorzugten Ausführungsform weist der Weg von der Einflussöffnung zur Abflussöffnung keine Verzweigungen auf.

Auf diese Weise ergibt sich ein einziger, durchgehender Weg durch alle Bereiche des Systems. Dies hat den Vorteil, dass sichergestellt wird, dass jeder Bereich des Systems stets durchflossen wird und somit eine Temperierung über das gesamte System verbessert wird.

Gemäß einer bevorzugten Ausführungsform ist die Einflussöffnung und/oder die Abflussöffnung an dem rechten, linken oder rückseitigen Abschnitt angebracht.

Eine Anbringung wenigstens einer der genannten Öffnungen an dem rechten, linken oder rückseitigen Abschnitt hat den Vorteil, dass die Verbindungsschläuche zu den betreffenden Öffnungen bei einer aufrechten Kopfhaltung entlang des Körpers herunter geführt werden können, wodurch die Belastungsspitzen auf den Verbindungsschläuchen reduziert werden. Da die betreffenden Öffnungen nicht am frontalen Abschnitt angebracht sind, wird außerdem vermieden, dass die Verbindungsschläuche vor dem Gesicht der Person herunterhängen oder dieses berühren.

Gemäß einer bevorzugten Ausführungsform weist wenigstens ein Abschnitt einen Trennsteg auf, welcher eingerichtet ist, das Temperierungsfluid durch den Abschnitt zu führen.

Durch derartige Trennstege können auf einfache Weise Kanäle für das Temperierungsfluid innerhalb eines Abschnitts geschaffen werden. Ein Trennsteg, welcher das Temperierungsfluid durch einen Abschnitt führt, hat den Vorteil, dass stets neu temperiertes Temperierungsfluid durch den Abschnitt vorteilhafter verteilt werden kann. Durch eine geringe Breite eines Trennstegs wird weiterhin die gesamte Fläche des Abschnitts temperiert.

Gemäß der Erfindung weist das System wenigstens ein Verbindungselement auf, welches eingerichtet ist, wenigstens zwei Abschnitte trennbar aneinander zu befestigen.

Unter einem "Verbindungselement" ist ein Element zu verstehen, welches ermöglicht, wenigstens zwei Abschnitte trennbar aneinander zu befestigen. Ein Verbindungselement ist jedoch im Allgemeinen nicht dazu bestimmt, eine fluidleitende Verbindung zwischen den beiden Abschnitten herzustellen. Ein Verbindungselement weist insbesondere einen Klettverschluss, eine Hakenverbindung und/oder eine Magnetverbindung auf.

Dies hat den Vorteil, dass das System zum Lagern eine im Wesentlichen ebene Form annehmen kann, in der das wenigstens eine Verbindungselement zwischen den beiden Abschnitten geöffnet ist und die Abschnitte dadurch "ausgebreitet" werden können, und beim Anlegen an den Kopf der Person durch Verbinden zweier Abschnitte mittels des wenigstens einen Verbindungselements von dieser im Wesentlichen ebenen Form in eine Form gebracht und in dieser fixiert werden kann, welche eine möglichst gute Kopfabdeckung ermöglicht, wodurch die Temperierung verbessert wird.

Gemäß der Erfindung ist die Form des Systems durch die trennbare Befestigung der wenigstens zwei Abschnitte aneinander durch das wenigstens eine Verbindungselement an die Form des Kopfes der Person anpassbar.

Dies hat den Vorteil, dass das System individuell an die Person angepasst werden kann, wodurch die Kopfoberfläche besser durch das System bedeckt wird und dadurch eine verbesserte Temperierung ermöglicht wird.

Weitere Vorteile, Merkmale und Anwendungsmöglichkeiten der vorliegenden Erfindung ergeben sich aus der nachfolgenden detaillierten Beschreibung im Zusammenhang mit den Figuren.

Dabei zeigt
**Fig. 1** schematisch einen Querschnitt durch den Hohlraum eines erfindungsgemäßen Systems zur Temperierung des Kopfes in ausgebreiteter Form parallel zur Oberseite und Unterseite des Systems;
**Fig. 2a** den Kopf einer Person während einer Anwendung des Systems von schräg vorne gesehen; und
**Fig. 2b** den Kopf einer Person während einer Anwendung des Systems von schräg hinten gesehen.

In den Figuren werden durchgängig dieselben Bezugszeichen für dieselben oder einander entsprechende Elemente der Erfindung verwendet.

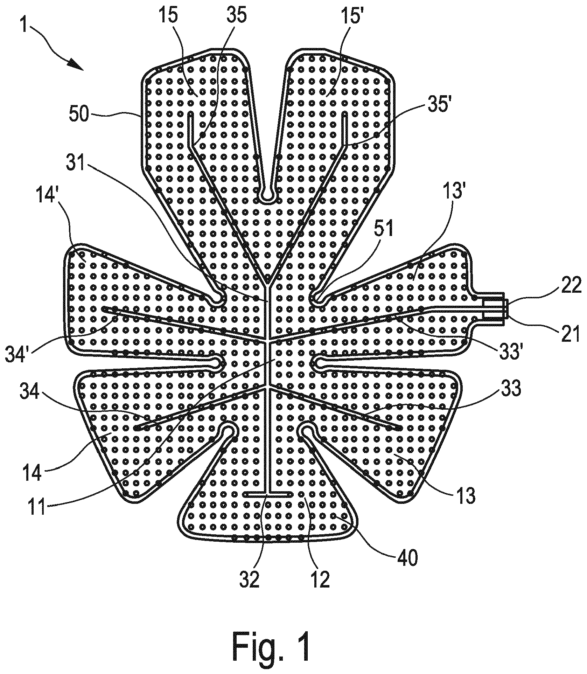

**Fig. 1** illustriert den Querschnitt eines erfindungsgemäßen Systems 1 zur Temperierung des Kopfes einer Person. Jeder Abschnitt besitzt eine Oberseite und eine Unterseite, vorzugsweise jeweils eine Kunststofffolie, sowie einen Hohlraum dazwischen, durch welchen das Temperierungsfluid fließen kann. Fig. 1 stellt einen Querschnitt parallel zu der Oberseite und der Unterseite durch den Hohlraum dar. Das dargestellte System 1 weist eine im Wesentlichen sternförmige Anordnung des frontalen Abschnitts 12, des linken Abschnitts mit den beiden linken Unterabschnitten 13, 13', des rechten Abschnitts mit den beiden rechten Unterabschnitten 14, 14' sowie des rückseitigen Abschnitts mit den beiden rückseitigen Unterabschnitten 15, 15' an dem zentralen Abschnitt 11 auf. Die Einflussöffnung 21 sowie die Abflussöffnung 22 sind an dem Unterabschnitt 13' des linken Abschnitts angebracht.

Der linke Unterabschnitt 13' weist einen Trennsteg 33' auf, welcher verhindert, dass das Temperierungsfluid direkt von der Einflussöffnung 21 zur Abflussöffnung 22 fließt, ohne vorher das gesamte System zu durchfließen. Auch die übrigen Abschnitte bzw. Unterabschnitte weisen entsprechende Trennstege auf. Der Trennsteg 33' führt das Temperierungsfluid von der Einflussöffnung 21 durch den linken Unterabschnitt 13' in einen Bereich des zentralen Abschnitts 11, der durch den Trennsteg 31 begrenzt wird, von welchem das Fluid in den linken Unterabschnitt 13 geleitet wird. Auf diese Art und Weise wird das Fluid in dieser Reihenfolge von jeweils einem der äußeren Abschnitte bzw. Unterabschnitte 13', 13, 12, 14, 14', 15, 15' in jeweils eine durch den Trennsteg 31 beschränkte Kammer des zentralen Abschnitts 11 geführt und von dort aus in den jeweils nächsten äußeren Abschnitt 13, 12, 14, 14', 15, 15', 13', bis das Fluid alle Abschnitte des Systems durchflossen hat und im linken Unterabschnitt 13' zur Abflussöffnung 22 gelangt.

Der Umfang des Systems wird durch eine Außenwand 50 begrenzt. Die Außenwand 50 wird im Ausführungsbeispiel durch eine umlaufende Schweißnaht der Oberseite mit der der Unterseite gebildet. An den Verbindungsstellen zwischen dem zentralen Abschnitt 11 und einem jeweiligen äußeren Abschnitt 12, 13, 13', 14, 14', 15, 15' finden sich Verbindungsrundungen 51 in der Außenwand 50, welche ermöglichen, dass die äußeren Abschnitte flexibler zueinander bewegt werden können, um eine vorteilhafte Anpassung an die Kopfform zu ermöglichen. Die Verbindungsrundungen 51 verringern außerdem etwaige Kerbwirkungen, die bei einer Bewegung der einzelnen äußeren Abschnitte gegeneinander entstehen können.

Über alle Abschnitte sind Abstandsnoppen 40 verteilt, welche verhindern, dass die Oberseite und die Unterseite "zusammenkleben" und dadurch flächig aneinander anliegen. Die Abstandsnoppen 40 sorgen auf diese Weise dafür, dass der Hohlraum des jeweiligen Abschnitts des Systems 1 eine festgelegte Höhe besitzt, um den Flusswiderstand des Systems 1 zu minimieren. Weiter verhindern gleichmäßig und im richtigen Abstand platzierte Abstandsnoppen 40 ein Bilden von Blasensäcken durch ein Anstauen des Temperierungsfluids. Solche Blasensäcke sorgen für eine starke Materialbeanspruchung direkt an den Abstandsnoppen 40 durch rechtwinklige Kraftzugvektoren im Material und somit ein erhöhtes Riss- und/oder Platzrisiko des Systems 1. Weiter stehen die Blasen aus der möglichst glatten und gleichmäßigen Oberfläche hervor, was ein gleichmäßiges Anliegen des Systems 1 am Kopf verhindert und dadurch zu einer schlechterer Temperierung des Kopfes führt. Die Abstandsnoppen 40 sind im Ausführungsbeispiel durch Schweißpunkte ausgeführt, durch welche die Oberseite mit der Unterseite verschweißt ist. Durch den geringen Querschnitt der Abstandsnoppen 40 wird auch mit Abstandsnoppen 40 die Oberfläche des Systems flächendeckend temperiert. Somit stellen die Abstandsnoppen 40 keine Abzweigung des Fluidflusses dar.

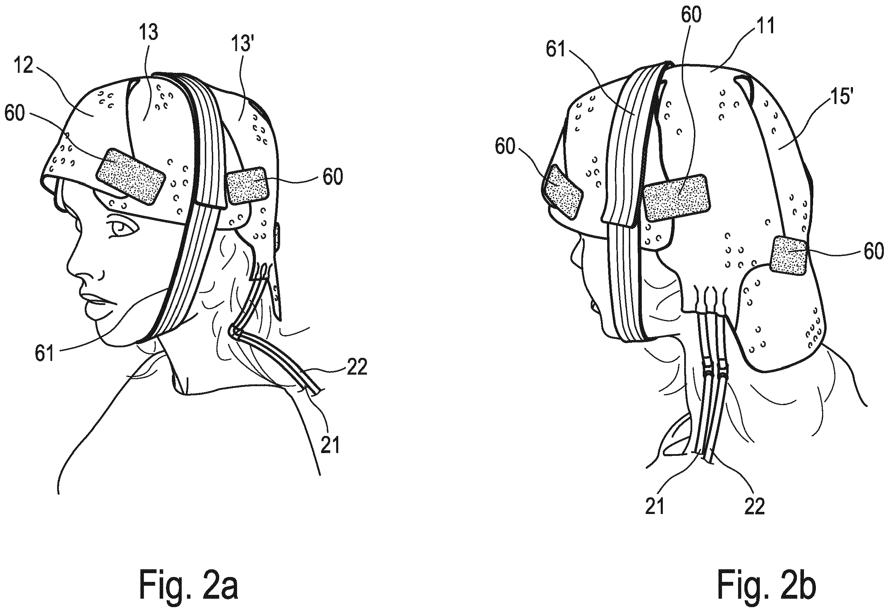

**Fig. 2a und Fig. 2b** illustrieren den Kopf einer Person während einer Anwendung des Systems 1. Zur Anwendung des Systems wird der zentrale Abschnitt 11 auf die Kopfoberseite der Person gelegt, während der frontale Abschnitt 12 auf die Stirn, der linke Unterabschnitt 13 auf die linke Schläfe, der linke Unterabschnitt 13' auf die linke Kopfseite, der rechte Unterabschnitt 14 auf die rechte Schläfe, der rechte Unterabschnitt 14' auf die rechte Kopfseite, der rechte rückseitige Unterabschnitt 15 auf die rechte Seite des Hinterkopfes und der linke rückseitige Unterabschnitt 15' auf die linke Seite des Hinterkopfes gelegt wird.

Die Lage der äußeren Abschnitte wird an die Kopfform angepasst, und die äußeren Abschnitte werden in dieser Form durch Verbindungselemente 60 aneinander fixiert. Ein Befestigungselement 61 fixiert das gesamte System an dem Kopf. Die Verbindungs- bzw. Befestigungselemente 60, 61 sind im Ausführungsbeispiel Bänder mit Klettverschlüssen.

### BEZUGSZEICHENLISTE

- 1: System zur Temperierung des Kopfes
- 11: Zentraler Abschnitt
- 12: Frontaler Abschnitt
- 13, 13': Unterabschnitte des linken Abschnitts
- 14, 14': Unterabschnitte des rechten Abschnitts
- 15, 15': Unterabschnitte des rückseitigen Abschnitts
- 21: Einflussöffnung
- 22: Abflussöffnung
- 31: Trennstegsystem im zentralen Abschnitt
- 32: Trennstegsystem im frontalen Abschnitt
- 33, 33': Trennstegsysteme in den Unterabschnitten des linken Abschnitts
- 34, 34': Trennstegsysteme in den Unterabschnitten des rechten Abschnitts
- 35, 35': Trennstegsysteme in den Unterabschnitten des rückseitigen Abschnitts
- 40: Abstandsnoppen
- 50: Außenwand
- 51: Verbindungsrundung
- 60: Verbindungselement
- 61: Befestigungselement

## Patentansprüche

1. System (1) zur Temperierung des Kopfes einer Person, aufweisend:
einen zentralen Abschnitt (11), welcher eingerichtet ist, wenigstens einen Teil der Oberseite des Kopfes zu bedecken;
wenigstens einen frontalen Abschnitt (12), welcher eingerichtet ist, wenigstens einen Teil der Stirn der Person zu bedecken;
wenigstens einen rechten Abschnitt (14, 14'), welcher eingerichtet ist, wenigstens einen Teil der rechten Seite des Kopfes der Person zu bedecken;
wenigstens einen linken Abschnitt (13, 13'), welcher eingerichtet ist, wenigstens einen Teil der linken Seite des Kopfes der Person zu bedecken; und
wenigstens einen rückseitigen Abschnitt (15, 15'), welcher eingerichtet ist, wenigstens einen Teil des Hinterkopfes der Person zu bedecken;
wobei jeder einzelne Abschnitt (11, 12, 13, 13', 14, 14', 15, 15') eingerichtet ist, von einem Temperierungsfluid, welches eingerichtet ist, den Kopf zu temperieren, durchflossen zu werden;
wobei jeder einzelne Abschnitt (11, 12, 13, 13', 14, 14', 15, 15') mit wenigstens einem weiteren Abschnitt (11, 12, 13, 13', 14, 14', 15, 15') verbunden ist und in einer Fluidverbindung für das Temperierungsfluid mit diesem weiteren Abschnitt (11, 12, 13, 13', 14, 14', 15, 15') steht;
wobei wenigstens ein Abschnitt (11, 12, 13, 13', 14, 14', 15, 15') eine Einflussöffnung (21) und wenigstens ein Abschnitt (11, 12, 13, 13', 14, 14', 15, 15') eine Abflussöffnung (22) für das Temperierungsfluid aufweist, wobei das System (1) zur Temperierung wenigstens ein Verbindungselement (60) aufweist, welches eingerichtet ist, wenigstens zwei Abschnitte (11, 12, 13, 13', 14, 14', 15, 15') trennbar aneinander zu befestigen, und wobei die Form des Systems (1) durch die trennbare Befestigung der wenigstens zwei Abschnitte (11, 12, 13, 13', 14, 14', 15, 15') aneinander an die Form des Kopfes der Person anpassbar ist.
**dadurch gekennzeichnet, dass** der linke Abschnitt (13 13'), der rechte Abschnitt (14, 14') und der rückseitige Abschnitt (15, 15') wenigstens zwei zueinander bewegbare Unterabschnitte aufweist, welche, wie auch der frontale Abschnitt (12), jeweils mit dem zentralen Abschnitt (11) verbunden sind und in einer Fluidverbindung für das Temperierungsfluid mit dem zentralen Abschnitt (11) stehen, und wobei das System (1) eine im Wesentlichen sternförmige Anordnung des frontalen Abschnitts (12), des linken Abschnitts mit den beiden linken Unterabschnitten (13, 13'), des rechten Abschnitts mit den beiden rechten Unterabschnitten (14, 14') sowie des rückseitigen Abschnitts mit den beiden rückseitigen Unterabschnitten (15, 15') an dem zentralen Abschnitt (11) aufweist.

2. System (1) zur Temperierung nach Anspruch 1, **dadurch gekennzeichnet, dass** das Temperierungsfluid nach Eintritt durch die Einflussöffnung (21) auf dem Weg zur Abflussöffnung (22) jeden Abschnitt (11, 12, 13, 13', 14, 14', 15, 15') des Systems (1) zur Temperierung durchfließt.

3. System (1) zur Temperierung nach dem vorhergehenden Anspruch, **dadurch gekennzeichnet, dass** der Weg von der Einflussöffnung (21) zur Abflussöffnung (22) keine Verzweigungen aufweist.

4. System (1) zur Temperierung nach einem der vorhergehenden Ansprüche, **dadurch gekennzeichnet, dass** die Einflussöffnung (21) und/oder die Abflussöffnung (22) an dem rechten Abschnitt (14, 14'), an dem linken Abschnitt (13, 13') oder an dem rückseitigen Abschnitt (15, 15') angebracht ist.

5. System (1) zur Temperierung nach einem der vorhergehenden Ansprüche, **dadurch gekennzeichnet, dass** wenigstens ein Abschnitt (11, 12, 13, 13', 14, 14', 15, 15') einen Trennsteg (31, 32, 33, 33', 34, 34', 35, 35') aufweist, welcher eingerichtet ist, das Temperierungsfluid durch den Abschnitt (11, 12, 13, 13', 14, 14', 15, 15') zu führen.

## Claims

1. A system (1) for temperature control of the head of a person, comprising:
a central section (11) configured to cover at least a part of the top of the head; at least one frontal section (12) configured to cover at least a part of the forehead of the person;
at least one right section (14, 14') configured to cover at least a part of the right side of the head of the person;
at least one left section (13, 13') configured to cover at least a part of the left side of the head of the person; and
at least one rear section (15, 15') configured to cover at least a part of the back of the head of the person;
wherein each individual section (11, 12, 13, 13', 14, 14', 15, 15') is configured to be flowed through by a temperature control fluid configured to control the temperature of the head;
wherein each individual section (11, 12, 13, 13', 14, 14', 15, 15') is connected to at least one further section (11, 12, 13, 13', 14, 14', 15, 15') and is in fluid communication for the temperature control fluid with this further section (11, 12, 13, 13', 14, 14', 15, 15');
wherein at least one section (11, 12, 13, 13', 14, 14', 15, 15') comprises an inflow opening (21) and at least one section (11, 12, 13, 13', 14, 14', 15, 15') comprises an outflow opening (22) for the temperature control fluid, wherein the system (1) for temperature control comprises at least one connecting element (60) which is arranged to attach at least two sections (11, 12, 13, 13', 14, 14', 15, 15') to one another in a separable manner, and wherein the shape of the system (1) can be adapted to the shape of the head of the person by the separable attachment of the at least two sections (11, 12, 13, 13', 14, 14', 15, 15') to one another,
**characterized in that** the left section (13, 13'), the right section (14, 14') and the rear section (15, 15') comprises at least two subsections movable relative to each other, which, like the frontal section (12), are each connected to the central section (11) and are in fluid communication for the temperature control fluid with the central section (11), and wherein
the system (1) comprises a substantially star-shaped arrangement of the frontal section (12), of the left section with the two left subsections (13, 13'), of the right section with the two right subsections (14, 14'), and of the rear section with the two rear subsections (15, 15') at the central section (11).

2. System (1) for temperature control according to claim 1, **characterized in that** the temperature control fluid, after entering through the inflow opening (21), flows through each section (11, 12, 13, 13', 14, 14', 15, 15') of the system (1) for temperature control on its way to the outflow opening (22).

3. System (1) for temperature control according to the preceding claim, **characterized in that** the path from the inflow opening (21) to the outflow opening (22) comprises no branches.

4. System (1) for temperature control according to one of the preceding claims, **characterized in that** the inflow opening (21) and/or the outflow opening (22) is attached to the right section (14, 14'), to the left section (13, 13') or to the rear section (15, 15').

5. System (1) for temperature control according to one of the preceding claims, **characterized in that** at least one section (11, 12, 13, 13', 14, 14', 15, 15') comprises a separator (31, 32, 33, 33', 34, 34', 35, 35') which is arranged to guide the temperature control fluid through the section (11, 12, 13, 13', 14, 14', 15, 15').

## Revendications

1. Système (1) de thermorégulation de la tête d'une personne, présentant :
une section centrale (11), laquelle est configurée pour couvrir au moins une partie du côté supérieur de la tête ;
au moins une section frontale (12), laquelle est configurée pour couvrir au moins une partie du front de la personne ;
au moins une section droite (14, 14'), laquelle est configurée pour couvrir au moins une partie du côté droit de la tête de la personne ;
au moins une section gauche (13, 13'), laquelle est configurée pour couvrir au moins une partie du côté gauche de la tête de la personne ; et au moins une section arrière (15, 15'), laquelle est configurée pour couvrir au moins une partie de la partie postérieure inférieure de la tête de la personne ;
dans lequel chaque section (11, 12, 13, 13', 14, 14', 15', 15') est configurée pour être traversée par un fluide de thermorégulation qui est configuré pour thermoréguler la tête ;
dans lequel chaque section (11, 12, 13, 13', 14, 14', 15, 15') est reliée à au moins une autre section (11, 12, 13, 13', 14, 14', 15, 15') et est en communication fluidique pour le fluide de thermorégulation avec cette autre section (11, 12, 13, 13', 14, 14', 15, 15') ;
dans lequel au moins une section (11, 12, 13, 13', 14, 14', 15, 15') présente un orifice pour flux entrant (21) et au moins une section (11, 12, 13, 13', 14, 14', 15, 15') présente un orifice pour flux sortant (22) pour le fluide de thermorégulation, dans lequel le système (1) de thermorégulation présente au moins un élément de liaison (60), lequel est configuré pour fixer au moins deux sections (11, 12, 13, 13', 14, 14', 15,
15') l'une sur l'autre de manière séparable, et dans lequel la forme du système (1) peut être adaptée à la forme de la tête de la personne grâce à la fixation séparable des au moins deux sections (11, 12, 13, 13', 14, 14', 15, 15'), **caractérisé en ce que** la section gauche (13, 13'), la section droite (14, 14') et la section arrière (15, 15') présentent au moins deux sous-sections pouvant être déplacées l'une par rapport à l'autre, lesquelles, tout comme la section frontale (12), sont reliées respectivement à la section centrale (11) et sont en communication fluidique pour le fluide de thermorégulation avec la section centrale (11), et dans lequel le système (1) présente une disposition sensiblement en étoile de la section frontale (12), de la section gauche avec les deux sous-sections gauches (13, 13'), de la section droite avec les deux sous-sections droites (14, 14') ainsi que de la section arrière avec les deux sous-sections arrière (15, 15') sur la section centrale (11).

2. Système (1) de thermorégulation selon la revendication 1, **caractérisé en ce que** le fluide de thermorégulation traverse chaque section (11, 12, 13, 13', 14, 14', 15, 15') du système (1) de thermorégulation après être entré par l'orifice pour flux entrant (21) en cheminant vers l'orifice pour flux sortant (22).

3. Système (1) de thermorégulation selon la revendication précédente, **caractérisé en ce que** le trajet depuis l'orifice pour flux entrant (21) vers l'orifice pour flux sortant (22) ne présente aucune ramification.

4. Système (1) de thermorégulation selon l'une quelconque des revendications précédentes, **caractérisé en ce que** l'orifice pour flux entrant (21) et/ou l'orifice pour flux sortant (22) sont installés sur la section droite (14, 14'), sur la section gauche (13, 13') ou sur la section arrière (15, 15').

5. Système (1) de thermorégulation selon l'une quelconque des revendications précédentes, **caractérisé en ce qu'**au moins une section (11, 12, 13, 13', 14, 14', 15, 15') présente une nervure de séparation (31, 32, 33, 33', 34, 34', 35, 35'), laquelle est configurée pour guider le fluide de thermorégulation à travers la section (11, 12, 13, 13', 14, 14', 15, 15').
